# EUROPEAN PATENT APPLICATION

(11) **EP 4 749 266 A1**
(43) Date of publication of application: **27.05.2026**
(21) Application number: 24844729.4
(22) Date of filing: 19.07.2024
(51) Int. Cl.: G01N 21/27

(54) **OPTICAL FIBER SPECTROMETER AND IN-SITU WATER MEASUREMENT INSTRUMENT COMPRISING SAME**

(30) Priority: 21.07.2023 CN 202310899836; 21.07.2023 CN 202321928735 U
(71) Applicant: GLIT TECHNOLOGIES (SHENZHEN) PTE LTD, Shenzhen, Guangdong 518110 (CN)
(72) Inventor: LIU, Minyu, Shenzhen, Guangdong 518110 (CN); YUAN, Gaoqiang, Shenzhen, Guangdong 518110 (CN)
(74) Representative: Becker Kurig & Partner Patentanwälte mbB
(86) International application number: PCT/CN2024/106463
(87) International publication number: WO 2025/021038

(57) **Abstract**

The present application discloses an optical fiber spectrometer and an in-situ water measurement instrument thereof, which belongs to the technical field of optical analytical instruments, and solves the problem that the existing optical fiber spectrometers with the CT optical system or the M optical system cannot be used in applications due to limitations of geometric sizes. The optical fiber spectrometer comprises a housing, a slit module, a collimating mirror, a grating module, a focusing mirror module, a reflecting mirror module and a linear array photodetector module being assembled in the housing. The slit module, the collimating mirror, the grating module, the focusing mirror module, the reflecting mirror module and the linear array photodetector module are assembled in the housing. When it is used, the incident light passes through the slit module, the collimating mirror, the grating module, the focusing mirror module, the reflecting mirror module and the linear array photodetector module to form an optical system of spectral detection. The optical fiber spectrometer of the present invention not only enables an angle of the reflecting mirror module to be adjustable but also shrinks a planar layout of the optical system, making a shape of the optical fiber spectrometer slender and allowing the optical fiber spectrometer to be used in scenarios where assembled geometric sizes are limited.

## Description

### Field of invention

The present invention belongs to the technical field of optical analytical instruments, and particularly relates to an optical fiber spectrometer and an in-situ water measurement instrument thereof.

### Background art of the invention

At present, an optical system structure of a conventional optical fiber spectrometer in the market comprises a slit module, a collimating mirror, a grating, a focusing mirror and a CCD photodetector. An optical system of the conventional optical fiber spectrometer has a Czerny-Turner (CT) structure, and optical components thereof are arranged in a same plane. Although people can use small-size optical components to fabricate the conventional small-size optical fiber spectrometer with the CT optical system, it is difficult to make the spectrometer with a slender shape, due to a fact that compression of geometric sizes of such a spectrometer in a plane will be greatly limited. For some applications that requirements of use of a slender optical fiber spectrometer with small factor, such as in in-situ water measurement applications, e.g. a sealed cylindrical probe is entirely immersed in water, thus it is needed to use a slender optical fiber spectrometer, and such kind of conventional spectrometer with the CT optical system is difficult to meet actual requirements. Similarly, a conventional optical fiber spectrometer with the M optical system has a same drawback because optical components thereof are also arranged in a same plane.

Therefore, it is truly necessary to develop a slender optical fiber spectrometer with small factor for in-situ water quality measurement that can be used in different and immersed scenarios.

### Disclosure of the invention

The purpose of the present invention is to provide an optical fiber spectrometer and an in-situ water measurement instrument thereof, which solves the problems of the existing optical fiber spectrometers with the CT optical system and the M optical system which planar layouts of optical systems are relatively large, the optical components are arranged in a same plane, and application requirements using slender optical fiber spectrometers with small factor cannot be met.

To achieve the above purpose, a technical solution of the present invention is fulfilled as follows: the optical fiber spectrometer comprises a housing, a slit module, a collimating mirror, a grating module, a focusing mirror module, a reflecting mirror module and a linear array photodetector module being assembled in the housing. The slit module, the collimating mirror, the grating module, the focusing mirror module, the reflecting mirror module and the linear array photodetector module are assembled around the inner periphery of the housing. When it is used, the incident light passes through the slit module, the collimating mirror, the grating module, the focusing mirror module, the reflecting mirror module and the linear array photodetector module to form an optical system of the spectral detection.

In some embodiments, the slit module, the grating module, the focusing mirror module, the linear array photodetector module, the collimating mirror and the reflecting mirror module are sequentially assembled around the inner periphery of the housing; and when it is used, the incident light sequentially passes through the slit module, the collimating mirror, the grating module, the focusing mirror module, the reflecting mirror module and the linear array photodetector module to form the CT optical system.

In some embodiments, two reflecting mirror modules are provided; the slit module, the collimating mirror, the focusing mirror module, the linear array photodetector module, one of the reflecting mirror modules, the grating module and the other reflecting mirror module are sequentially assembled around the inner periphery of the housing; and when it is used, the incident light sequentially passes through the slit module, the other reflecting mirror module, the collimating mirror, the grating module, the focusing mirror module, one of the reflecting mirror modules and the linear array photodetector module to form the M optical system.

In some embodiments, the slit module comprises an optical fiber coupling connector and a slit, and the optical fiber coupling connector is used for fixing an optical fiber or a coupling lens.

In some embodiments, the grating module comprises a grating holder and a grating being assembled on the grating holder, and the grating holder is assembled into the housing; the grating holder comprises a base, a grating holder support and a rotating shaft; the grating holder support and the grating are assembled on the base, and the grating is closely attached to the grating holder support; the rotating shaft is designed at bottom of the base; and the rotating shaft is connected into the housing.

In some embodiments, top of the grating holder support is designed with an adjusting notch, and the base is designed with a positioning block that is closely attached to the grating; and the top of the grating holder support is also designed with fixing holes on both sides of the adjusting notch.

In some embodiments, the reflecting mirror module comprises a reflecting mirror holder and a reflecting mirror being assembled on the reflecting mirror holder, and the reflecting mirror holder is assembled into the housing.

In some embodiments, the reflecting mirror holder comprises a reflecting mirror holder body, a limiting block and a rotating shaft; the limiting block is fixedly arranged on side of the reflecting mirror holder body; the rotating shaft is located on side of the reflecting mirror holder body closing to the limiting block and is connected to the reflecting mirror holder body; and the rotating shaft is assembled into the housing.

In some embodiments, an upper edge of the reflecting mirror holder body is extended with a first positioning plate and a second positioning plate that are used for positioning the reflecting mirror, and the reflecting mirror is arranged in a space being comprised of the first positioning plate and the second positioning plate.

Another technical solution of the present invention is fulfilled as follows: the in-situ water measurement instrument comprises an in-situ water measurement probe housing, the optical fiber spectrometer, the xenon lamp, the control circuit, the transmitting sealing window, a receiving sealing window and an in-situ water measurement probe space; the optical fiber spectrometer, the xenon lamp and the control circuit are sealed and assembled in the in-situ water measurement probe housing; the transmitting sealing window is used for sealing a through hole of the in-situ water measurement probe housing on side of the xenon lamp; the receiving sealing window is used for sealing a through hole of the in-situ water measurement probe housing on side of the optical fiber spectrometer; the in-situ water measurement probe space is formed by the transmitting sealing window and the receiving sealing window; and the optical fiber spectrometer, the xenon lamp, the transmitting sealing window and the receiving sealing window are assembled on a same optical axis to allow the light to pass through.

Compared with the prior art, the optical fiber spectrometer of the present invention is different from the prior art in that the optical components are not arranged in a same plane, and a reflecting mirror module with an adjustable angle is added in the present invention, which greatly shrinks a planar layout of the optical system, making a shape of the optical fiber spectrometer slender and allowing the optical fiber spectrometer to be used in scenarios where assembled geometric sizes are limited. Therefore, the optical fiber spectrometer of the present invention can be applied to an immersion-type in-situ water measurement instrument for water quality measurements in different scenarios, such as measurements of surface water, groundwater and nearshore seawater, thus to fulfill inline water quality measurement without second time pollution.

### Description of the drawings

Fig. 1 is a schematic diagram of an optical fiber spectrometer with the CT optical system in embodiment 1 of the present invention;
Fig. 2 is a schematic diagram of an optical fiber spectrometer with the M optical system in embodiment 2 of the present invention;
Fig. 3 is a schematic diagram of a grating module in an embodiment of the present invention;
Fig. 4 is a schematic diagram of a reflecting mirror module in an embodiment of the present invention;
Fig. 5 is another schematic diagram of a reflecting mirror module in an embodiment of the present invention;
Fig. 6 is a schematic diagram of a linear array photodetector module in an embodiment of the present invention;
Fig. 7 is a schematic diagram of an in-situ water measurement instrument in embodiment 3 of the present invention;
Fig. 8 is a spectrum of an in-situ water measurement instrument in embodiment 3 of the present invention.

In the figures: 1. slit module, 2. collimating mirror, 3. grating module, 31. grating holder, 311. base, 312. grating holder support, 3121. adjusting notch, 3122. fixing hole, 32. grating, 4. focusing mirror module, 5. reflecting mirror module, 51. reflecting mirror holder, 511. reflecting mirror holder body, 5111. first positioning plate, 5112. second positioning plate, 512. limiting block, 52. reflecting mirror, 6. linear array photodetector module, and 61. light-sensitive surface of the linear array photodetector module.

### Detailed description of the invention

To make the purpose, the technical solution and the advantages of the present invention more clear, the present invention will be further described below in detail in combination with specific embodiments. It should be understood that the specific embodiments described herein are only used for explaining the present invention, not used for limiting the present invention.

It should be clear in the description of the present invention that terms such as "perpendicular", "transverse", "longitudinal", "front", "rear", "left", "right", "upper", "lower", "horizontal", etc. indicate direction or position relationships shown based on the drawings, and are only intended to facilitate the description of the present invention rather than to mean that the indicated device or element must have a specific direction or position, and therefore, shall not be understood as a limitation to the present invention. It should be noted in the description of the present invention that, unless otherwise specifically regulated and defined, terms such as "installation," "connected," and "connecting" shall be understood in broad sense, and for example, may refer to fixed connection or detachable connection or integral connection, may refer to mechanical connection or electrical connection, and may refer to direct connection or indirect connection through an intermediate medium. For those ordinary skilled in the art, the specific meanings of the above terms in the present invention may be understood according to specific conditions.

### Embodiment 1

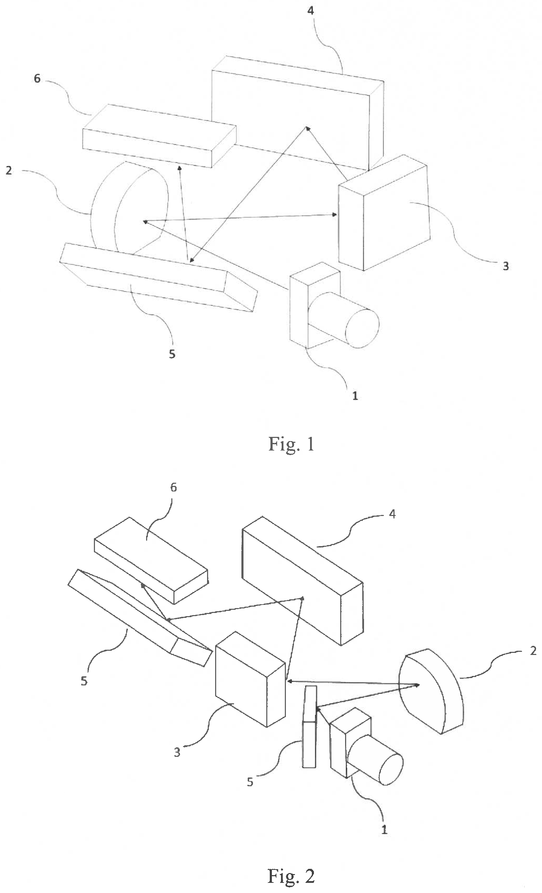

An optical fiber spectrometer provided in embodiment 1 of the present invention, as shown in Fig. 1, comprises a housing, a slit module 1, a collimating mirror 2, a grating module 3, a focusing mirror module 4, a reflecting mirror module 5 and a linear array photodetector module 6 being assembled in the housing. The slit module 1, the grating module 3, the focusing mirror module 4, the linear array photodetector module 6, the collimating mirror 2 and the reflecting mirror module 5 are sequentially assembled around the inner periphery of the housing; and when it is used, the incident light sequentially passes through the slit module 1, the collimating mirror 2, the grating module 3, the focusing mirror module 4, the reflecting mirror module 5 and the linear array photodetector module 6 to form the CT optical system.

After the above solution is adopted, the optical fiber spectrometer of the present invention is different from the prior art in that the optical components are not arranged in a same plane, and positions of internal optical components are adjustable as needed, which reduces a volume of the optical fiber spectrometer and makes the optical fiber spectrometer slender. The optical fiber spectrometer of the present invention can be applied to the immersion-type in-situ water measurement instrument for water quality measurements in different scenarios, such as measurements of surface water, groundwater and nearshore seawater, thus to fulfill inline water quality measurement without second time pollution.

In a specific implementation process of the present embodiment, the slit module 1 comprises an optical fiber coupling connector and a slit, and the optical fiber coupling connector is used for fixing an optical fiber or a coupling lens.

More specifically, the slit module 1 is comprised of an optical fiber coupling connector and a slit, the optical fiber coupling connector is used for fixing an optical fiber or a coupling lens, and measured light is conducted into the slit module 1 by the optical fiber or the coupling lens. A thin linear light is formed after the measured light passing through the slit, emitting towards the collimating mirror 2; the thin linear light is collimated towards the grating module 3 by the collimating mirror 2; a continuous distribution of different monochromatic lights in space is formed and conducted towards the focusing mirror module 4 with diffractive dispersion of the collimated light by the grating module 3; the continuous distribution of different monochromatic lights in space is focused towards the reflecting mirror module 5 by the focusing mirror module 4; and then the continuous distribution of different monochromatic lights in space is reflected towards and focused on the linear array photodetector module 6 by the reflecting mirror module 5 at a certain angle, such as 45 degrees, to form a linear and continuous distribution of different monochromatic lights, and a measured spectrum in form of electric signal is output with photoelectric conversion by the linear array photodetector module 6.

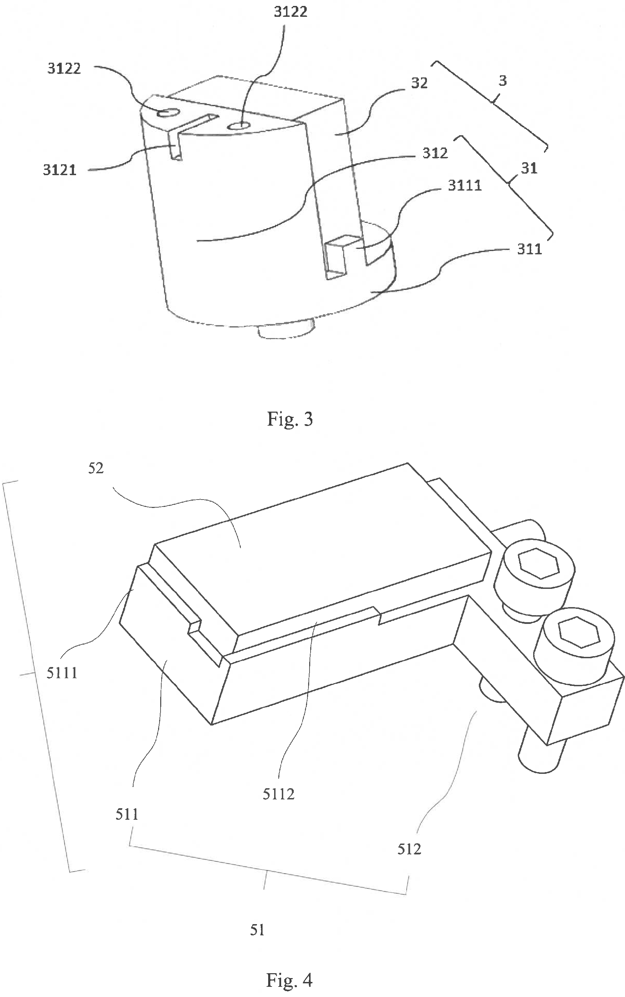

In the specific implementation process of the present embodiment, as shown in Fig. 3, the grating module 3 comprises a grating holder 31 and a grating 32 being assembled on the grating holder 31, and the grating holder 31 is assembled into the housing; the grating holder 31 comprises a base 311, a grating holder support 312 and a rotating shaft; the grating holder support 312 and the grating 32 are assembled on the base 311, and the grating 32 is closely attached to the grating holder support 312; the rotating shaft is designed at bottom of the base 311; and the rotating shaft is connected into the housing.

In the specific implementation process of the present embodiment, as shown in Fig. 3, top of the grating holder support 312 is designed with an adjusting notch 3121, and the base 311 is designed with a positioning block 3111 that is closely attached to the grating 32; and the top of the grating holder support 312 is also designed with fixing holes 3122 on both sides of the adjusting notch 3121.

More specifically, the grating 32 is fixed on the grating holder 31 through the positioning block 3111, a tool is used to adjust rotation of the grating holder 31 with the adjusting notch 3121, the rotation of the grating holder 31 is fulfilled around the rotating shaft, the rotating shaft is inserted into a hole of a folding optical fiber spectrometer with the CT optical system with tight fit, and the rotation of the grating holder 31 is used for adjusting a position of spectrum in the X-axis direction of the linear array photodetector module 6 (i.e., a direction in which pixels of the linear array photodetector module 6 are sequentially arranged). After adjustment of the grating holder 31 is completed, screws are used to fix the grating holder 31 into the folding optical fiber spectrometer with the CT optical system through the fixing holes 3122 on both sides of the adjusting notch 3121. In the present embodiment, fixation is not limited to use the screws, bolts or pins, and other fitting methods can also be used for the fixation.

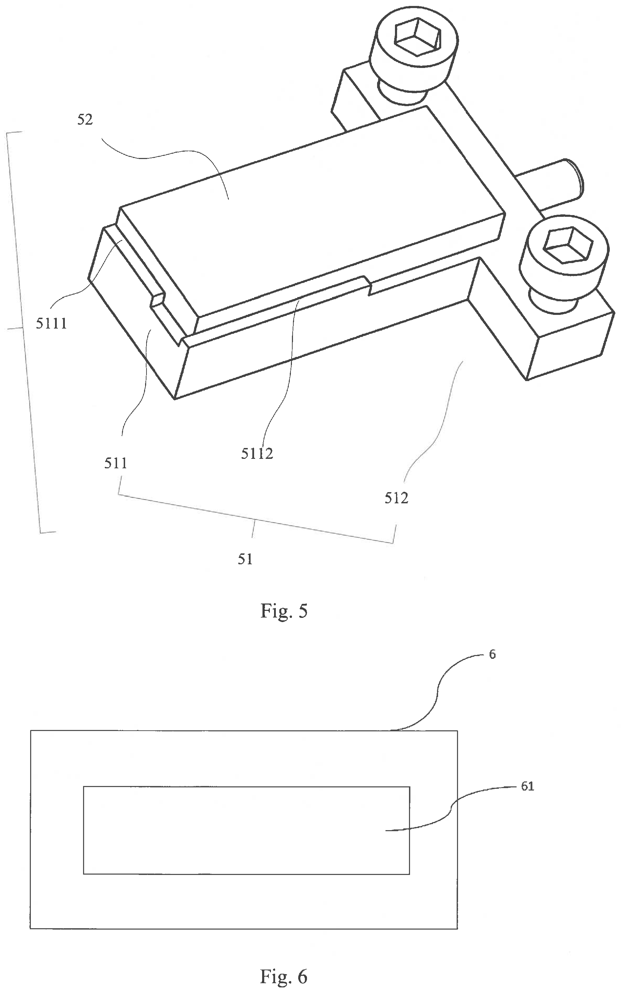

In the specific implementation process of the present embodiment, as shown in Fig. 4 and Fig. 5, the reflecting mirror module 5 comprises a reflecting mirror holder 51 and a reflecting mirror 52 being assembled on the reflecting mirror holder 51, and the reflecting mirror holder 51 is assembled into the housing.

In the specific implementation process of the present embodiment, as shown in Fig. 4 and Fig. 5, the reflecting mirror holder 51 comprises a reflecting mirror holder body 511, a limiting block 512 and a rotating shaft; the limiting block 512 is fixedly arranged on side of the reflecting mirror holder body 511; the rotating shaft is located on side of the reflecting mirror holder body 511 closing to the limiting block 512 and is connected to the reflecting mirror holder body 511; and the rotating shaft is assembled into the housing.

In the specific implementation process of the present embodiment, as shown in Fig. 4 and Fig. 5, an upper edge of the reflecting mirror holder body 511 is extended with a first positioning plate 5111 and a second positioning plate 5112 that are used for positioning the reflecting mirror 52, and the reflecting mirror 52 is arranged in a space being comprised of the first positioning plate 5111 and the second positioning plate 5112.

More specifically, the reflecting mirror 52 is fixed on the reflecting mirror holder 51, and the reflecting mirror 52 is arranged in a space being comprised of the first positioning plate 5111 and the second positioning plate 5112, and is positioned in two directions in a same plane by the first positioning plate 5111 and the second positioning plate 5112. Rotation of the reflecting mirror holder 51 is fulfilled by adjusting the limiting block 512 to adjust a position of spectrum in the Y-axis direction of the linear array photodetector module 6 (i.e., perpendicular to the direction in which the pixels of the linear array photodetector module 6 are sequentially arranged).

Further, as shown in Fig. 4, the limiting block 512 and the reflecting mirror holder body 511 are in L shape; a through hole and a screw hole are sequentially designed in the limiting block 512; the limiting block 512 is assembled to the housing through two connecting components; the connecting components are screws and can also be other components; one connecting component is put into the through hole that is tightened to fit a screw hole in the housing and thus to provide a fastening force; in addition, the other connecting component is put in the screw hole that is tightened to contact with the housing and thus to provide a pushing force. Rotation and locking fixation of the reflecting mirror holder 51 are fulfilled by adjusting tightness of the two connecting components, the reflecting mirror holder 51 is rotated around the rotating shaft, and the rotating shaft is inserted into the hole of the optical fiber spectrometer with the CT optical system with tight fit.

Further, as shown in Fig. 5, the limiting block 512 and the reflecting mirror holder body 511 are in T shape; two through holes are symmetrically designed in the limiting block 512; the limiting block 512 is connected to the housing through two connecting components; the connecting components are screws and can also be other components; the two connecting components are put into the two through holes; and the two connecting components are tightened alternately to fit screw holes in the housing. Rotation and locking fixation of the reflecting mirror holder 51 are fulfilled by adjusting tightness of the two connecting components, the reflecting mirror holder 51 is rotated around the rotating shaft, and the rotating shaft is inserted into the hole of the optical fiber spectrometer with the CT optical system with tight fit.

In the specific implementation process of the present embodiment, as shown in Fig. 6, a light-sensitive surface of the linear array photodetector module 61 is arranged in the linear array photodetector module 6.

More specifically, a light-sensitive surface of the linear array photodetector module 61 is arranged in the linear array photodetector module 6, and the light-sensitive surface of the linear array photodetector module 61 has the X-axis in a horizontal direction and the Y-axis in a vertical direction. The linear array photodetector module 6 can be a device, such as a CCD, CMOS or APD array. The light-sensitive surface of the linear array photodetector module 61 can be comprised of pixels of different sizes, such as 256, 512, 1024 and 2048 pixels. Focused measured light is imaged on the light-sensitive surface of the linear array photodetector module 61, which is perpendicular to the Y-axis in the vertical direction of the light-sensitive surface of the linear array photodetector module 61, to form the linear and continuous distribution of different monochromatic lights along the X-axis in the horizontal direction of the light-sensitive surface of the linear array photodetector module 61, and a measured spectrum (i.e., distribution of light intensity with the pixels of the light-sensitive surface of the linear array photodetector module 61) in form of electric signal with photoelectric conversion by the linear array photodetector module 6 can be output. If a wavelength of the optical fiber spectrometer of the present invention is calibrated by a standard lamp, the spectrum can also be expressed as distribution of the light intensity vs. the wavelength.

A work flow provided by embodiment 1 of the present invention is as follows: a thin linear light is formed after the measured light passing through the slit, emitting towards the collimating mirror 2; the thin linear light is collimated towards the grating module 3 by the collimating mirror 2; a continuous distribution of different monochromatic lights in space is formed and conducted towards the focusing mirror module 4 with diffractive dispersion of the collimated light by the grating module 3; rotation of the grating holder 31 is adjusted with the adjusting notch 3121 to adjust a position of spectrum in the X-axis direction of the linear array photodetector module 6; the continuous distribution of different monochromatic lights in space is focused towards the reflecting mirror module 5 by the focusing mirror module 4; rotation of the reflecting mirror holder 51 is fulfilled by adjusting the limiting block 512 to adjust a position of spectrum in the Y-axis direction of the linear array photodetector module 6; and then the continuous distribution of different monochromatic lights in space is reflected towards and focused on the linear array photodetector module 6 by the reflecting mirror module 5 at a certain angle, such as 45 degrees, to form a linear and continuous distribution of different monochromatic lights, with an optical system being formed as the CT optical system, and a measured spectrum in form of electric signal is output with photoelectric conversion by the linear array photodetector module 6.

### Embodiment 2

An optical fiber spectrometer provided in embodiment 2 of the present invention, as shown in Fig. 2, is provided with two reflecting mirror modules 5; the slit module 1, the collimating mirror 2, the focusing mirror module 4, the linear array photodetector module 6, one of the reflecting mirror modules 5, the grating module 3 and the other reflecting mirror module 5 are sequentially assembled around the inner periphery of the housing; and when it is used, the incident light sequentially-passes through the slit module 1, the other reflecting mirror module 5, the collimating mirror 2, the grating module 3, the focusing mirror module 4, one of the reflecting mirror modules 5 and the linear array photodetector module 6 to form the M optical system.

Embodiment 2 is different from embodiment 1 in that embodiment 2 is designed with two reflecting mirror modules 5; the measured light is conducted into the slit module 1 by the optical fiber or the coupling lens; a thin linear light is formed after the measured light passing through the slit, emitting towards one reflecting mirror module 5 at an angle of 45 degrees, is reflected towards the collimating mirror 2 at an angle of 45 degrees by the reflecting mirror module 5 at an angle of 45 degrees, and then is collimated towards the grating module 3 by the collimating mirror 2; a continuous distribution of different monochromatic lights in space is formed and conducted towards the focusing mirror module 4 with diffractive dispersion of the collimated light by the grating module 3; the continuous distribution of different monochromatic lights in space is focused towards the other reflecting mirror module 5 by the focusing mirror module 4; and then the continuous distribution of different monochromatic lights in space is reflected towards and focused on the linear array photodetector module 6 by the other reflecting mirror module 5 at a certain angle, such as 45 degrees, to form a linear and continuous distribution of different monochromatic lights, and a measured spectrum in form of electric signal is output with photoelectric conversion by the linear array photodetector module 6.

A work flow provided by embodiment 2 of the present invention is as follows: a thin linear light is formed after the measured light passing through the slit, emitting towards one reflecting mirror module 5 at an angle of 45 degrees, is reflected towards the collimating mirror 2 at an angle of 45 degrees by the reflecting mirror module 5 at an angle of 45 degrees, and then is collimated towards the grating module 3 by the collimating mirror 2; a continuous distribution of different monochromatic lights in space is formed and conducted towards the focusing mirror module 4 with diffractive dispersion of the collimated light by the grating module 3; rotation of the grating holder 31 is adjusted with the adjusting notch 3121 to adjust a position of spectrum in the X-axis direction of the linear array photodetector module 6; the continuous distribution of different monochromatic lights in space is focused towards the other reflecting mirror module 5 by the focusing mirror module 4; rotation of the reflecting mirror holder 51 is fulfilled by adjusting the limiting block 512 to adjust a position of spectrum in the Y-axis direction of the linear array photodetector module 6; and then the continuous distribution of different monochromatic lights in space is reflected towards and focused on the linear array photodetector module 6 by the other reflecting mirror module 5 at a certain angle, such as 45 degrees, to form a linear and continuous distribution of different monochromatic lights, with an optical system being formed as the M optical system, and a measured spectrum in form of electric signal is output with photoelectric conversion by the linear array photodetector module 6.

### Embodiment 3

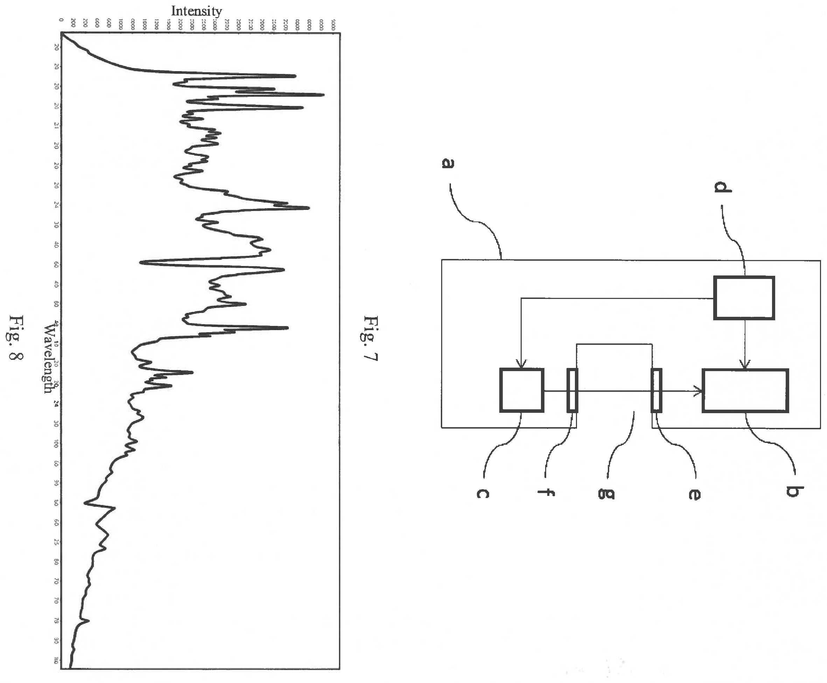

Embodiment 3 of the present invention provides an in-situ water measurement instrument, as shown in Fig. 7. In Fig. 7, a is an in-situ water measurement probe housing, b is the optical fiber spectrometer in embodiments 1 and 2, c is a xenon lamp, d is a control circuit, e is a receiving sealing window, f is a transmitting sealing window, and g is an in-situ water measurement probe space; the in-situ water measurement instrument comprises the in-situ water measurement probe housing a, the optical fiber spectrometer b in embodiments 1 and 2, the xenon lamp c, the control circuit d, the receiving sealing window e, the transmitting sealing window f and the in-situ water measurement probe space g; the optical fiber spectrometer b, the xenon lamp c and the control circuit d are sealed and assembled in the in-situ water measurement probe housing a; the transmitting sealing window f is used for sealing a through hole of the in-situ water measurement probe housing a on side of the xenon lamp c; the receiving sealing window e is used for sealing a through hole of the in-situ water measurement probe housing a on side of the optical fiber spectrometer b; the in-situ water measurement probe space g is formed by the transmitting sealing window f and the receiving sealing window e; and the optical fiber spectrometer b, the xenon lamp c, the transmitting sealing window f and the receiving sealing window e are assembled on a same optical axis to allow light to pass through.

More specifically, the optical fiber spectrometer b, the xenon lamp c and the control circuit d of the present invention are sealed and fixed in the in-situ water measurement probe housing a; the transmitting sealing window f is used for sealing a through hole of the in-situ water measurement probe housing a on side of the xenon lamp c and ensuring that measuring light passes through; the receiving sealing window e is used for sealing a through hole of the in-situ water measurement probe housing a on side of the optical fiber spectrometer b in embodiments 1 and 2 of the present invention and ensuring that measuring light passes through; and the in-situ water measurement probe space g is formed by the transmitting sealing window f and the receiving sealing window e to ensure that a measuring optical system is immersed in water measured. Broad-spectrum measuring light including ultraviolet light, visible light and near infrared light emitted by the xenon lamp c passes through the transmitting sealing window f and enters the water in the in-situ water measurement probe space g; the measuring light absorbed by the water enters the optical fiber spectrometer b in embodiments 1 and 2 of the present invention after passing through the receiving sealing window e; an absorption spectrum of the water is measured by the optical fiber spectrometer b; and composition, concentration etc. of the water measured can be calculated after being compared with a pre-stored reference spectrum.

The optical fiber spectrometer with the M optical system in embodiment 2 is used in embodiment 3 to measure a spectrum of the xenon lamp, ranging from 200 nanometers to 820 nanometers, as shown in Fig. 8. It can be seen that the spectrum covers spectra involved in in-situ water measurement, including 220 nanometers and 275 nanometers for measuring total nitrogen, 600 nanometers for measuring chemical oxygen demand (COD), 660 nanometers for measuring ammonia nitrogen, 700 nanometers for measuring total phosphorus, etc.

A work flow provided by embodiment 3 of the present invention is as follows: broad-spectrum measuring light including ultraviolet light, visible light and near infrared light emitted by the xenon lamp c passes through the transmitting sealing window f and enters water in the in-situ water measurement probe space g; the measuring light absorbed by the water enters the optical fiber spectrometer b in embodiments 1 and 2 of the present invention after passing through the receiving sealing window e; an absorption spectrum of the water is measured by the optical fiber spectrometer b; and composition and concentration of the water measured can be calculated after being compared with a pre-stored reference spectrum, thus to fulfill inline water quality measurement without second time pollution.

To sum up, the optical fiber spectrometer of the present invention is different from the prior art in that optical components are not located in a same plane, addition of the reflecting mirror module in the present invention not only enables an angle of the focused spectral beam to be adjustable but also shortens the optical system, and positions of internal optical components are adjustable as needed, which makes the optical fiber spectrometer compact in size. The optical fiber spectrometer of the present invention can be applied to an in-situ water measurement instrument for water quality measurements in different scenarios, such as measurements of surface water, groundwater and nearshore seawater, thus to fulfill inline water quality measurement without second time pollution.

The above only describes preferred specific embodiments of the present invention, but the protection scope of the present invention is not limited thereto. Any change or replacement contemplated easily by those skilled in the art familiar with the technical field within the technical scope disclosed by the present invention shall be covered within the protection scope of the present invention. Therefore, the protection scope of the present invention should be determined by the protection scope of the claims.

## Claims

1. An optical fiber spectrometer, **characterized in** comprising a housing, a slit module (1), a collimating mirror (2), a grating module (3), a focusing mirror module (4), a reflecting mirror module (5) and a linear array photodetector module (6) being assembled in the housing; the slit module (1), the collimating mirror (2), the grating module (3), the focusing mirror module (4), the reflecting mirror module (5) and the linear array photodetector module (6) are assembled around the inner periphery of the housing; and when it is used, the incident light passes through the slit module (1), the collimating mirror (2), the grating module (3), the focusing mirror module (4), the reflecting mirror module (5) and the linear array photodetector module (6) to form an optical system of the spectral detection.

2. The optical fiber spectrometer as claimed in claim 1, **characterized in that** the slit module (1), the grating module (3), the focusing mirror module (4), the linear array photodetector module (6), the collimating mirror (2) and the reflecting mirror module (5) are sequentially assembled around the inner periphery of the housing; and when it is used, the incident light sequentially passes through the slit module (1), the collimating mirror (2), the grating module (3), the focusing mirror module (4), the reflecting mirror module (5) and the linear array photodetector module (6) to form the CT optical system.

3. The optical fiber spectrometer as claimed in claim 1, **characterized in that** two reflecting mirrors modules (5) are provided; the slit module (1), the collimating mirror (2), the focusing mirror module (4), the linear array photodetector module (6), one of the reflecting mirror modules (5), the grating module (3) and the other reflecting mirror module (5) are sequentially assembled around the inner periphery of the housing; and when it is used, the incident light sequentially passes through the slit module (1), the other reflecting mirror module (5), the collimating mirror (2), the grating module (3), the focusing mirror module (4), one of the reflecting mirror modules (5) and the linear array photodetector module (6) to form the M optical system.

4. The optical fiber spectrometer as claimed in any one of claims 1-3, **characterized in that** the slit module (1) comprises an optical fiber coupling connector and a slit, and the optical fiber coupling connector is used for fixing an optical fiber or a coupling lens.

5. The optical fiber spectrometer as claimed in claim 4, **characterized in that** the grating module (3) comprises a grating holder (31) and a grating (32) being assembled on the grating holder (31), and the grating holder (31) is assembled into the housing; the grating holder (31) comprises a base (311), a grating holder support (312) and a rotating shaft; the grating holder support (312) and the grating (32) are assembled on the base (311), and the grating (32) is closely attached to the grating holder support (312); the rotating shaft is designed at bottom of the base (311); and the rotating shaft is connected into the housing.

6. The optical fiber spectrometer as claimed in claim 5, **characterized in that** top of the grating holder support (312) is designed with an adjusting notch (3121), and the base (311) is designed with a positioning block (3111) that is closely attached to the grating (32); and the top of the grating holder support (312) is also designed with fixing holes (3122) on both sides of the adjusting notch (3121).

7. The optical fiber spectrometer as claimed in any one of claims 1-3, **characterized in that** the reflecting mirror module (5) comprises a reflecting mirror holder (51) and a reflecting mirror (52) being assembled on the reflecting mirror holder (51), and the reflecting mirror holder (51) is assembled into the housing.

8. The optical fiber spectrometer as claimed in claim 7, **characterized in that** the reflecting mirror holder (51) comprises a reflecting mirror holder body (511), a limiting block (512) and a rotating shaft; the limiting block (512) is fixedly arranged on side of the reflecting mirror holder body (511); the rotating shaft is located on side of the reflecting mirror holder body (511) closing to the limiting block (512) and is connected to the reflecting mirror holder body (511); and the rotating shaft is assembled into the housing.

9. The optical fiber spectrometer as claimed in claim 8, **characterized in that** an upper edge of the reflecting mirror holder body (511) is extended with a first positioning plate (5111) and a second positioning plate (5112) that are used for positioning the reflecting mirror (52), and the reflecting mirror (52) is arranged in a space being comprised of the first positioning plate (5111) and the second positioning plate (5112).

10. An in-situ water measurement instrument, **characterized in** comprising an in-situ water measurement probe housing, the optical fiber spectrometer as claimed in any one of claims 1-9, a xenon lamp, a control circuit, a transmitting sealing window, a receiving sealing window and an in-situ water measurement probe space; the optical fiber spectrometer, the xenon lamp and the control circuit are sealed and assembled in the in-situ water measurement probe housing; the transmitting sealing window is used for sealing a through hole of the in-situ water measurement probe housing on side of the xenon lamp; the receiving sealing window is used for sealing a through hole of the in-situ water measurement probe housing on side of the optical fiber spectrometer; the in-situ water measurement probe space is formed by the transmitting sealing window and the receiving sealing window; and the optical fiber spectrometer, the xenon lamp, the transmitting sealing window and the receiving sealing window are assembled on a same optical axis to allow the light to pass through.
